# EUROPEAN PATENT SPECIFICATION

(11) **EP 3 221 811 B1**
(45) Date of publication and mention of the grant of the patent: **08.04.2020**
(21) Application number: 15797358.7
(22) Date of filing: 20.11.2015
(51) Int. Cl.: G16H 10/60, G16H 50/30, G16H 40/40, H04R 25/00

(54) **METHOD AND SYSTEM FOR ESTABLISHING NETWORK CONNECTION TO A WEARABLE EEG MONITORING MODULE**
VERFAHREN UND SYSTEM ZUM AUFBAU EINER NETZWERKVERBINDUNG ZU EINEM AM KÖRPER TRAGBAREN EEG-ÜBERWACHUNGSMODUL
PROCÉDÉ ET SYSTÈME POUR ÉTABLIR UNE CONNEXION RÉSEAU AVEC UN MODULE DE SURVEILLANCE D'ÉLECTROCARDIOGRAMME (EEG) POUVANT ÊTRE PORTÉ

(30) Priority: 20.11.2014 WO PCT/EP2014/075111
(43) Date of publication of application: 27.09.2017
(73) Proprietor: T&W Engineering A/S, 3540 Lynge (DK)
(72) Inventor: WESTERMANN, Soren Erik, DK-3540 Lynge (DK); ANDERSEN, Svend Vitting, DK-3540 Lynge (DK); WESTERGAARD, Anders, DK-3540 Lynge (DK); MARETTI, Niels Erik Boelskift, DK-3540 Lynge (DK)
(86) International application number: PCT/EP2015/077210
(87) International publication number: WO 2016/079294

(56) References cited:
- EP-A1- 2 760 225
- WO-A1-03/043494
- WO-A2-2014/182638
- US-A1- 2009 058 635
- US-B1- 8 882 666

## Description

The invention relates to a system and a method for providing a network connection to a wearable EEG monitoring module via the Internet. More specifically the invention relates to a system for remote surveillance of an EEG signal of a person susceptible of having a hypoglycemic seizure, where the system comprises an EEG sensor having electrodes for measuring one or more EEG signals from said person. The invention also relates to a wearable EEG monitoring module.

For persons with diabetes accurate control of blood sugar concentration is important. The level should not be too high in order to limit the risk of long term effects of diabetes. The blood sugar level should also not be too low, since this might lead to hypoglycemia, where the person becomes absent and may become unconscious. Hypoglycemic attacks may be fatal. Often persons with diabetes have the problem that they will not feel any warning before the blood sugar concentration has fallen to a level where a hypoglycemic attack occurs. This phenomenon is known as hypoglycemic unawareness. The risk of an attack therefore often limits the possible activities of the people concerned, which furthermore decreases their quality of life. Attacks can be prevented in a simple way, e.g. by consuming appropriate food when glucose values become critically low. The number of people in the risk group is approximately 10 million.

A large part of people with diabetes are elderly persons, of which some cannot always be trusted to take the necessary actions themselves on an alarm of an imminent hypoglycemic attack. The same may be the case for children having diabetes. For persons who may need assistance in preventing an imminent hypoglycemic attack it is necessary to have a system which can also transmit an alarm to a helper, e.g. caregiver, who is not necessarily in the same room. In the case of children the helper would typically be a parent. In order to avoid errors it is essential that the handling of such a system is as simple as possible, and that it is easy to control the system. Simple handling includes that intervals between changing or recharging batteries should be as long as possible. Easy control of the system includes that it should be easy to check correct positioning of the part arranged on the skin surface of the body. Simple handling and easy control is especially important in a nursing home where one helper or caregiver may need to watch out for more than one alarm system related to different persons and at the same time be doing a number of other tasks.

WO03043494 A1 discloses a cluster system for remote monitoring and diagnostic support. The sensors are arranged in a cluster for interactive communication. The system forms a local communication network having access to Internet and is adapted for wireless, remote, real-time two-way data communication between the system units or through Internet. The two-way data communication is adapted for entering of instructions for acquiring and interpreting data from a number of data sources.

EP2760225 A1 discloses a distributed, location-based system for facilitating connectivity of hearing instruments. US 2009/058635 A1 discloses facilitating the access of monitored health related data by remote devices which are used by health-care personnel.

The purpose of the invention is to provide remote access to wearable EEG monitoring modules allowing medical care professionals to retrieve logging data from the wearable EEG monitoring modules for analyses and to adjust settings for the wearable EEG monitoring modules.

The scope of the invention is determined by the appending independent claims. Preferable embodiments are determined by the appending dependent claims.

The invention will be described in further detail with reference to preferred aspects and the accompanying drawing, in which:
fig. 1 illustrates schematically a system for providing a network connection to a wearable EEG monitoring module according to a first embodiment of the invention;
fig. 2 illustrates in more details the system shown in fig 1;
fig. 3 illustrates schematically a system for providing a network connection to a wearable EEG monitoring module according to a second embodiment of the invention;
fig. 4 illustrates in more details the system shown in fig 3;
fig. 5 illustrates schematically the data exchange in the network connection system according to an embodiment of the invention;
fig. 6 shows a flowchart for accessing a wearable EEG monitoring module over the Internet according to an embodiment of the invention;
fig. 7 shows a flowchart for maintaining the access data for a wearable EEG monitoring module according to an embodiment of the invention; and
fig. 8 shows a flowchart of a method for handling the establishment of a direct connection to a wearable EEG monitoring module according to an embodiment of the invention.

### DETAILED DESCRIPTION

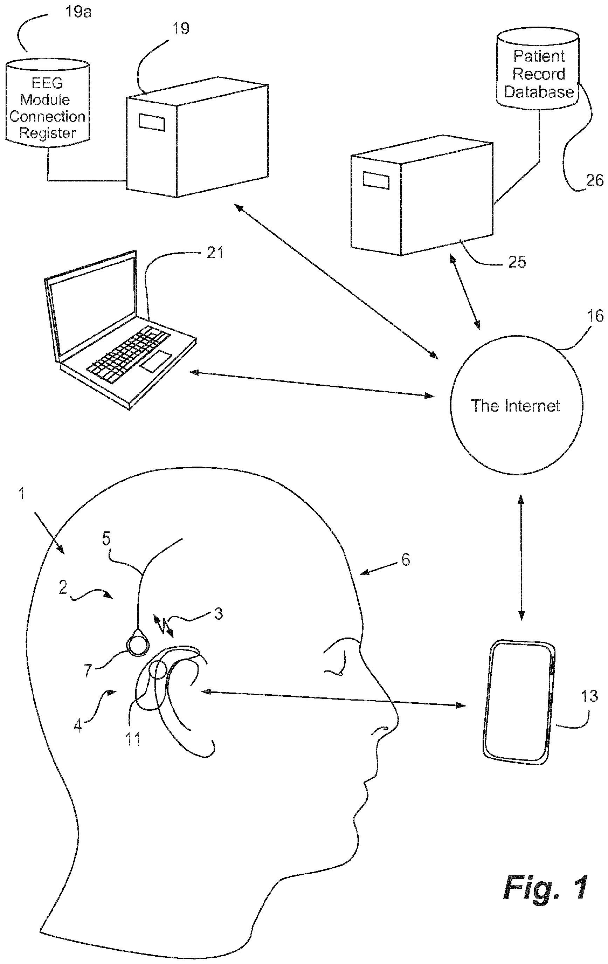

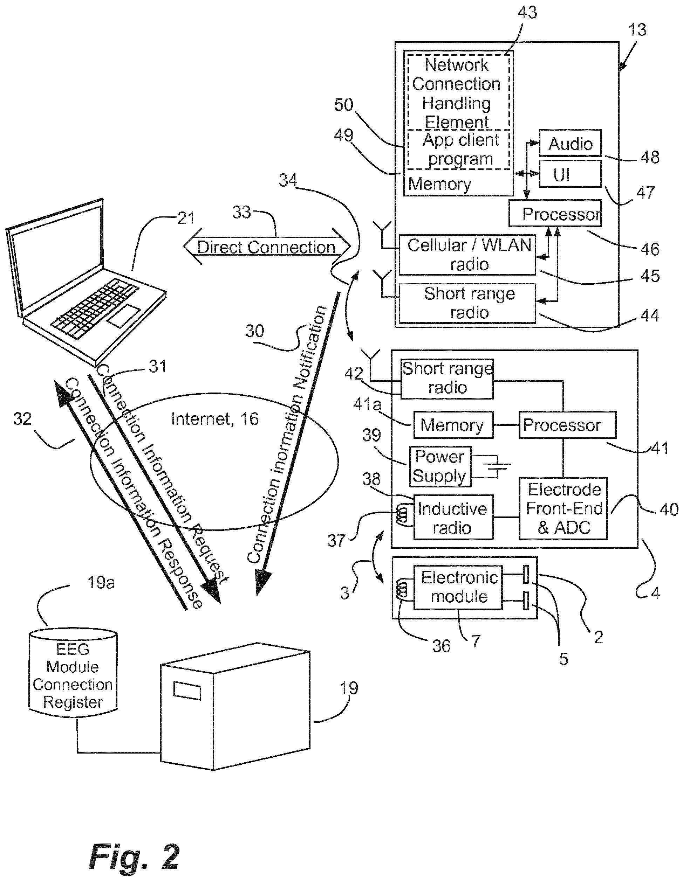

Reference is made to fig. 1 and 2, which schematically illustrates a system for providing a network connection for a computer device 21 to wearable EEG monitoring module 1 having a gateway the Internet 16. A gateway is a network node that allows a network to interface with another network with different protocols. A head 6 of a person provided with a wearable EEG monitoring module 1 according to a first embodiment of the invention is shown. The wearable EEG monitoring module 1 is adapted for remote surveillance of an EEG signal. The person being monitored wears the wearable EEG monitoring module 1 comprising an implantable EEG sensor 2 and an associated processing unit 4. The implantable EEG sensor 2 and the processing unit 4 are adapted to be in wireless communication 3 through the skin of the person. The implanted EEG sensor 2 comprises electrodes 5. The EEG sensor 2 will have at least two electrodes 5, which may be arranged as separate electrodes along the same wire as illustrated. One wire comprising all electrodes associated with respective conductors may facilitate the implantation process. The EEG sensor 2 is adapted for implantation on the head. This implantation may be subcutaneous or intra cranial. The advantage is that a better contact between electrodes and tissue can be obtained. A subcutaneous EEG sensor can also be implanted relatively easy.

The implantable EEG sensor 2 is provided with an electronic module 7 receiving one or more differential EEG signals from at least two electrodes 5, and delivering an output signal via a coil 36. The processing unit 4 is preferably arranged at the ear of the person of whom the EEG signal is being monitored. Preferably, the processing unit 4 is arranged in a housing behind the ear as a behind the ear hearing aid. This also facilitates a position as close as possible to the implanted part, which is important for the wireless communication and power transfer through the skin. With the position behind the ear of the housing, means for fixation is also necessary. For this purpose part of a wire or sound tubing also applied for conveying the sound from processing unit 4 into the ear canal may be used. This means that this wire or sound tubing should be relatively stiff, i.e. adapted for keeping the original given shape during use. Further to this purpose the wire or sound tubing can also be used for providing a sound alarm or message to the user. In the case of a wire the loudspeaker or receiver could be arranged within or close to the ear canal. In the case of sound tubing the loudspeaker or receiver could be arranged in the housing of the processing unit.

The wearable EEG monitoring module 1 communicates preferably with the personal communication device 13 - here shown as a smartphone. The personal communication device 13 according to the invention is Internet enabled which means that the personal communication device 13 may access the Internet 16 via a wireless Internet connection (e.g. WLAN such as 802.11_{a, b or g}), or a cellular data connection (e.g. WCDMA or LTE). Advantageously, the personal communication device 13 has the ability to download and launch application software from an app store via the Internet. The term "app" is short for application software which is a set of one or more programs designed to carry out operations for a specific application. Application software cannot run on itself but is dependent on system software to execute.

A server 25 hosts a centrally operated outpatient Electronic Medical Record (EMR) system or an electronic patient medical database 26 being accessible over the Internet; and each patient record involving remote surveillance of an EEG signal includes a unique identity for an associated EEG monitoring module 1. Furthermore, a control server 19 is providing an EEG module connection register 19a including unique identity for EEG modules with associated connection information, preferably including the IP address and port number. The personal communication device 13 includes a network connection handling element 43 which in the embodiment shown in fig. 2, which is not pursued by the present invention, is being adapted for detecting the presence of the short range wireless connection 34 between the wearable EEG monitoring module 1 and the gateway. The network connection handling element 43 is integrated as a part of the personal communication device 13, and is adapted for uploading current connection information to the control server 19. The control server 19 is adapted for updating the connection information in the EEG Module connection register 19a when receiving uploaded connection information. The control server 19 furthermore provides the connection information to a computer device 21 upon request. The computer device 21 is adapted for establishing a direct connection to the at least one EEG monitoring module 1 based on the connection information requested from the control server 19.

The wireless connection 3 between the implantable EEG sensor 2 and the processing unit 4 is based on an inductive coupling between the coil 36 and a coil 37 in an inductive radio 38 of the processing unit 4. This wireless connection 3 is applied to transfer power from the processing unit 4 to the implantable EEG sensor 2. Thereby, it is possible to operate the implantable EEG sensor 2 without a battery. The two coils 36, 37 should be closely aligned on each side of the skin barrier in order to achieve an efficient power transfer. The center axis of one coil should preferably continue through the center axis of the other coil, or the two center axes should be arranged close to each other. This close arrangement is ensured when arranging the processing unit. The implantable EEG sensor 2 must be placed such during implantation that it will be easy to align the two coils, i.e. the implantable EEG sensor 2 should be arranged in a position where its inductive coil is at a position where it will be easy to arrange the coil of the processing unit 4 aligned. In case of insufficient alignment between the two coils for obtaining a satisfactory power and data transmission, this can be detected by the processing unit 4 and a notification can be sent to the personal communication device 13.

The wireless connection based on inductive coupling is also applied for transmission of the EEG signal from the implanted EEG sensor 2 to the processing unit 4. This may be done by varying the load on the coil of implanted EEG sensor 2. The changes in load in the implanted EEG sensor 2 influence the power load on the coil in the processing unit 4. In this way an EEG signal can be transmitted from the implanted EEG sensor 2 to the processing unit 4. The inductive radio 38 switches between two modes - in the first mode the power supply powers the implanted EEG sensor 2, and in the second mode implanted EEG sensor 2 transmits the differential EEG signal to the inductive radio 38 which forwards the data via a module 40 operating as electrode front-end and Analog to Digital Converter (ADC) to a digital signal controller 41. The processing unit 4 comprises a power supply 39 including a battery, e.g. a standard hearing aid battery, for powering the processing unit 4 and the implantable EEG sensor 2 via the wireless connection 3. The processing unit 4 also comprises a digital signal controller 41 for analyzing the EEG signal according to predefined algorithms in order to identify an imminent seizure. The processing unit 4 comprises a memory 41a, such as an EEPROM, for logging EEG signals, specific events or alarms. The processing unit 4 includes a short range radio 42 for communication with the personal communication device 13.

A short range radio 44 connects the personal communication device 13 to the wearable EEG monitoring module 1 by means of the Bluetooth Low Energy protocol. The personal communication device 13 has memory 49 (e.g. EEPROM) - in which a control app 50 for the wearable EEG monitoring module 1 is stored. The personal communication device 13 is adapted to present the control app 50 on a touch screen to the user. The control app 50 is adapted to offer control elements, display alarm (together with an acoustic alarm), and present logged data.

The control app 50 of the personal communication device 13 hosts the network connection handling element 43. The personal communication device 13 is according the present embodiment a smartphone. The personal communication device 13 includes a processor 46 controlling the operation of audio elements 48 (like a speaker and a microphone) and user interface (UI) elements 47 (like a touch screen or keys and a display). Furthermore the processor 46 controls the operation of a transceiver 45 serving various mobile communication standards and WLAN standards connecting the personal communication device 13 to the Internet 16. The transceiver 45 may establish one or more competing Internet connections, e.g. via the cellular network and via a public hotspot or a router at home. The network connection handling element 43 identifies the current connection information, preferably including the IP address and port number, for the Internet connection identified. The network connection handling element 43 detects the presence of the short range communication channel between the personal communication device 13 and the wearable EEG monitoring module 1, and the long range communication channel from the personal communication device 13 to the Internet 16.

The connection information includes the current IP address of the EEG monitoring module 1. Furthermore, the connection information may also include the port number of the EEG monitoring module 1, as an IP address is always associated with a port of the host. The port is the endpoint of communication in the hosts operating system. In some situations it may be advantageous to identify the protocol type of the communication in the connection information.

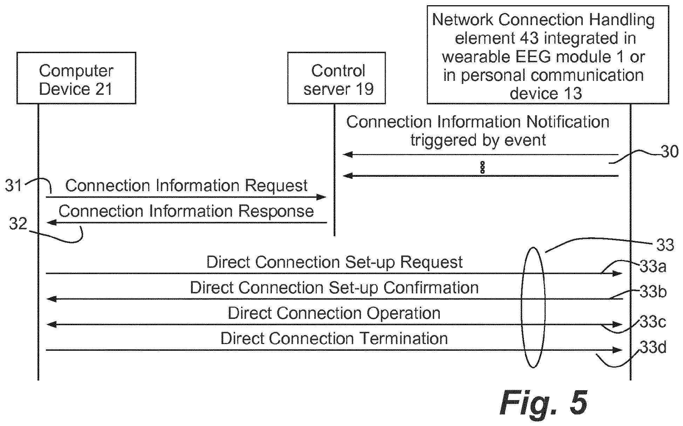

When the short range communication channel and long range communication channel both are present, the network connection handling element 43 transmits a connection information notification 30 (also shown in fig. 5) over the Internet 16 to the control server 19 whose connection information is preprogrammed into the network connection handling element 43. The connection information notification 30 provides the unique identity for the EEG monitoring module 1 linked to the current Internet addresses and preferably also port number.

The transmission of the connection information notification 30 is trigged by an event - both the short range communication channel and long range communication channel are present. Hereby the control server 19 is able to maintain its EEG module connection register 19a as a look-up table in which the unique identity for the wearable EEG monitoring module 1 may be translated to the current connection information of the network connection handling element 43. The connection information in EEG module connection register 19a for a unique identity for the wearable EEG monitoring module 1 may be prioritized according to data bandwidth or data pricing, and these information may be provided by the personal communication device 13.

The network connection handling element 43 serves several purposes - it monitors status of the connectivity for the wearable EEG monitoring module 1, handles session security, and notifies the current connection information to an EEG module connection register 19a. The network connection handling element 43 may be downloaded to the personal communication device 13 from an app store and stored in a memory, e.g. Solid State Drive, of the personal communication device 13.

When a medical professional or a monitoring server needs to exchange or collect data from the wearable EEG monitoring module 1, the patient medical record may be accessed in the patient record server 25 from where the unique identity for the EEG monitoring module in question may be retrieved. Preferably, the program handling the data exchange and retrieving the unique identity for the EEG monitoring module 1, automatically sends the connection information request 31 to the control server 19, and sets up the direct connection 33, preferably using UDP hole punching, to the network connection handling element 43 based on the connection information response 32 from the EEG module connection register 19a. The direct connection 33 is handled as described with reference to fig. 5.

The centrally operated outpatient Electronic Medical Record (EMR) system or an electronic patient medical database 26 contains personal information such as name, address and additional contact data like phone number and e-mail address. For each patient, the record contains data like birthday, social security number, and health insurance number. For each patient record there is provided security elements as credentials for accessing to one or more data fields and secure keys for establishing a secure connection between the computer device 21 and the wearable EEG monitoring module 1. The patient may by means of the stored security elements edit credentials via a web-interface. The patient record manager may by means of certificates control persons allowed to Read and Edit data in the patient record and thereby in the wearable EEG monitoring module 1.

Finally the patient record includes a data field identifying the wearable EEG monitoring module 1 associated with the account, and identified by manufacturer, EEG monitoring module model, serial number, and software version.

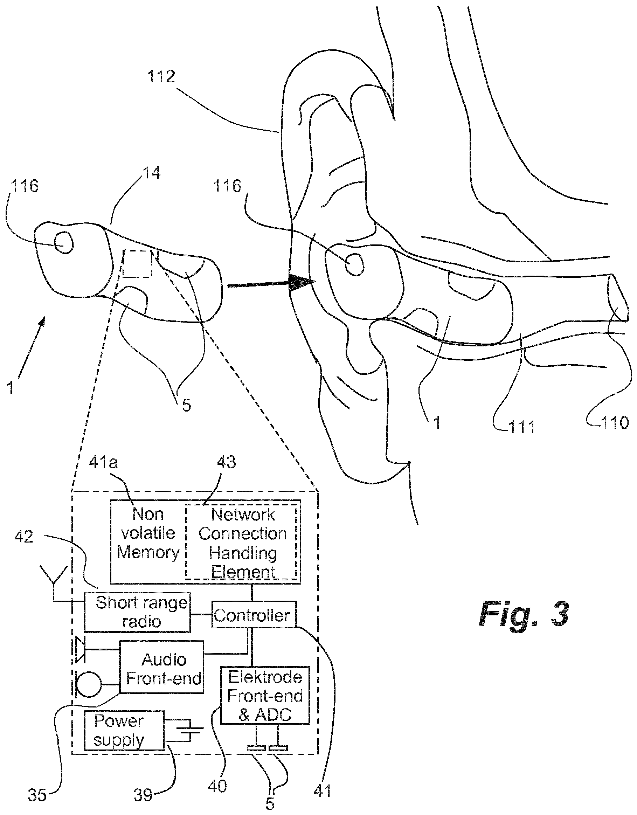

Fig. 3 shows a wearable EEG monitoring module 1 according to one aspect of the invention. The EEG monitoring module 1 can be worn in the ear canal of a person to be monitored, e.g. for detecting hypoglycemia, e.g. like a per se known In-The-Canal (ITC) hearing aid. Furthermore, the device will allow healthcare personal to remote monitor and record EEG data for the patient - either continuously or as periodical upload of logged data (data harvesting). Healthcare personnel would then be allowed to monitor patients who have regularly recurring problems like seizures or micro-sleep. The wearable EEG monitoring module 1 will not interfere with normal life, because the wearable EEG monitoring module 1 has an acoustic vent 116 so the wearer will be able to hear. After a while, the wearer forgets that he wears the wearable EEG monitoring module 1. The wearable EEG monitoring module 1 is on its outer surface provided with two active electrodes 5. Internally the wearable EEG monitoring module 1 contains a customized housing 14. The EEG monitoring module 1 is arranged in the ear canal with the electrodes in contact with the tissue in the ear canal. Thereby any kind of surgery is avoided, but the contact between the electrodes and the tissue is less stable.

The wearable EEG monitoring module 1 in the customized housing 14 is formed to fit into the external auditory canal 111 of the wearer, and defines a cavity in the external auditory canal 111 together with the tympanic membrane 110, and the cavity is opened by means of the acoustic vent 116 extending through the entire length of the wearable EEG monitoring module 1. Preferably the wearable EEG monitoring module 1 does not extend beyond the pinna 112.

The electronic components of the wearable EEG monitoring module 1 are shown schematically in enlarged view in the dotted box. The electronic components include a power supply 39 based upon a standard hearing aid battery for powering the electronics. The two electrodes 5 provided on the surface of the wearable EEG monitoring module 1 pick up a potential and delivers the data via a module 40 operating as electrode frontend and Analog to Digital Converter (ADC) to a controller 41. The controller 41 receives the amplified and digitized signal for processing. The controller 41 analyses the EEG signal picked up for detecting hypoglycemia by monitoring the brain wave frequency, and if the brain wave frequency falls beyond a predefined interval, this may indicate that a medical emergency may arise. Hypoglycemia is a medical emergency that involves an abnormally diminished content of glucose in the blood.

Upon detection of abnormal brain wave activities, the controller 41 provides an alert for the patient via an audio front-end module 35 including a microphone and a speaker. With the microphone, the controller 41 is able to pick up audio samples and classify the current sound environment. Furthermore, the controller 41 establishes a wireless communication link by means of a short range radio 42 being able to connect to the Internet 16 via a communication standard, such as the Bluetooth™ Low Energy standard. The controller 41 adjusts the predefined interval for normal brain wave activity in correlation to the real time clock information and the sound environment classification. With the speaker, the controller 41 is able to alert the wearer of the wearable EEG monitoring module 1 that a medical emergency may arise and that precautionary actions have to be taken.

The controller 41 is connected to a memory 41a, such as an EEPROM, for logging EEG signals, specific events or alarms. The memory 41a further hosts the network connection handling element 43, and this will be described in details below.

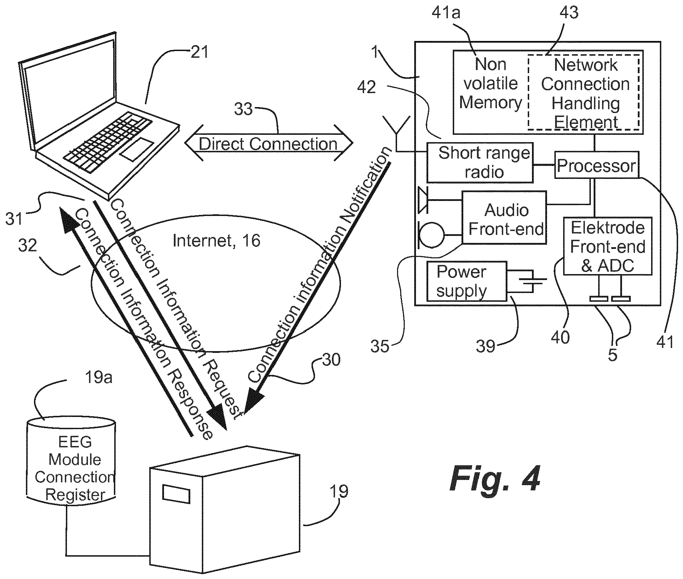

Referring to fig. 4, in which it is seen that the EEG monitoring module 1 comprises the electronic components shown in fig. 3. The EEG monitoring module 1 uses short range radio 42 for establishing a wireless connection to a gateway to the Internet 16. This gateway may be the personal communication device 13, a router (e.g. WLAN) in a private domain or a hotspot in a public domain. A hotspot is a physical location that offers Internet access over a wireless local area network (WLAN) through the use of a router connected to a link to an Internet service provider. Hotspots typically use Wi-Fi technology.

The EEG monitoring module 1 comprises a network connection handling element 43 stored as a software program in the non-volatile memory 41a. The network connection handling element 43 detects when a new network connection to the EEG monitoring module 1 has been established - e.g. a connection to a hotspot or to a router in the private domain, and when the current connection information has been identified, the network connection handling element 43 transmits a connection information notification 30 over the Internet 16 to the control server 19. The address of the control server 19 is advantageously preprogrammed into the network connection handling element 43. The connection information notification 30 provides a unique identity for the EEG monitoring module 1 linked to its current connection information. The transmission of the connection information notification 30 is trigged by an event, e.g. the establishment of a connection to the Internet via a gateway. Hereby the control server 19 is able to maintain its EEG module connection register 19a as a look-up table in which the unique identity for the EEG monitoring module 1 may be translated to the current connection information of the EEG monitoring module 1.

The Internet is adapted to relay datagrams across network boundaries based on the Internet Protocol (IP). The routing function according to the Internet protocol enables internetworking, and essentially establishes the Internet. The Internet Protocol has the task of delivering packets from the source host to the destination host solely based on the IP addresses in the packet headers. For this purpose, IP defines packet structures that encapsulate the data to be delivered. It also defines addressing methods that are used to label the datagram with both source and destination IP addresses. When sending the connection information notification 30, the current connection information of the network connection handling element 43 is present as source address of the data packet embodying the connection information notification 30, and the unique identity for the EEG monitoring module 1 is contained in the data packet. The control server 19 extracts these two elements and updates the EEG module connection register 19a accordingly.

The network connection handling element 43 serves several purposes - it monitors status of the connectivity of the EEG monitoring module 1, handles session security, and notifies the current connection information to the EEG module connection register 19a. The network connection handling element 43 is embedded into the EEG monitoring module before it leaves the factory, and some elements may be stored in the non-volatile memory 41a and some elements as security elements and certificates in a non-editable memory.

The invention is advantageous when a medical care professional, during a remote EEG surveillance for minimizing the risk of an imminent hypoglycemic seizure, analyzes log data from the wearable EEG monitoring module 1 - either in response to an alarm or in a scheduled session where the patient data are analyzed regularly in order to detect anomaly. This could be data or information on incidences where an alarm has almost been triggered but was prevented. Prevention of an alarm could take place if the person under EEG surveillance was eating or drinking, thereby increasing the blood sugar concentration, just before the alarm would have been triggered. Also data indicating the physical status of the person, e.g. sleeping or awake, could be transferred.

The remote EEG surveillance includes manipulation of a remote wearable EEG monitoring module 1, and this manipulation may include remote control of the wearable EEG monitoring module 1 - e.g. remote switching between day, night or exercise mode for the module; data logging of data captured by the wearable EEG monitoring module 1; and setting of operational parameters for EEG sensing, e. g. thresholds for the alarm function.

When a medical care professional needs to manipulate the setting of the EEG monitoring module 1, he may via the computer device 21 access the patient record from the patient record server 25 from where he may retrieve the unique identity for the wearable EEG monitoring module 1 in question. Preferably the surveillance software running on the computer device 21 automatically sends a connection Information request 31 from the account based on the unique identity for the wearable EEG monitoring module 1 in question to the control server 19. Based upon a look-up in the EEG Module Connection Register 19a, the control server 19 responds to the connection Information request 31 by sending a connection information response 32 containing the current connection information of the wearable EEG monitoring module 1 based on the unique identity stored in the patient record.

The computer device 21 then starts to set up a direct connection 33 preferably using UDP (User Datagram Protocol) hole punching (used for setting up connections between Internet hosts in private networks using network address translators) for establishing a bidirectional UDP connection. The computer device 21 sends a direct connection set-up request 33a to the network connection handling element 43, and if the EEG monitoring module 1 is still on-line, the network connection handling element 43 confirms the request by sending a direct connection set-up confirmation 33b. Hereafter, the computer device 21 and the network connection handling element 43 start to exchange payload in a bi-directional data packet exchange or direct connection operation 33c. The payload may include upload of data from the EEG monitoring module 1, such as logging data and settings, and download of data to the EEG monitoring module 1, such as instruction for remote control of the wearable EEG monitoring module 1. The computer device 21 disconnects the direct connection by sending a direct connection termination instruction 33d to the network connection handling element 43 once the exchange of data packets have been successfully completed.

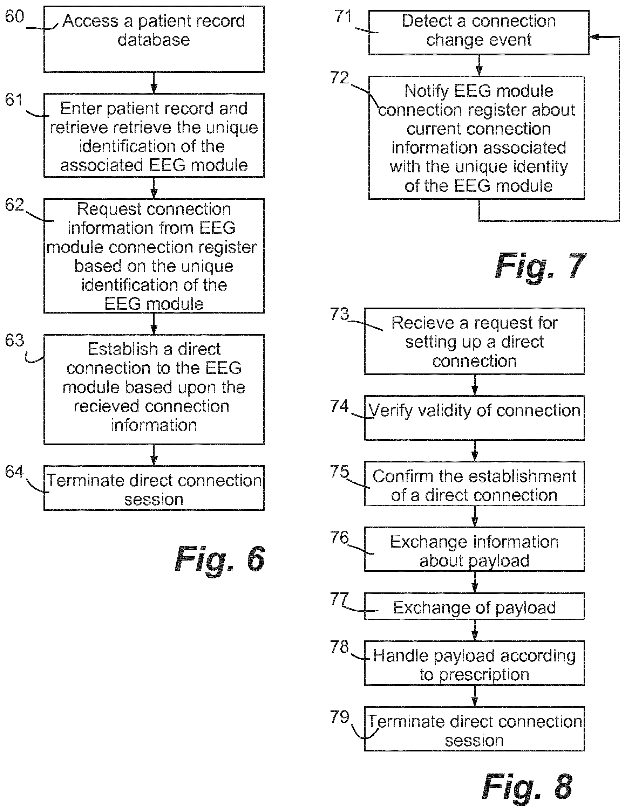

Fig. 6 illustrates a flowchart of a method for accessing an EEG monitoring module 1 over the Internet 16 according to an embodiment of the invention. An entity, e.g. a computer (M2M - "machine-to-machine" - data consolidation) or a medical care professional via a computer 21, intending to connect to the EEG monitoring module 1, accesses in step 60 the patient record database 26. If the entity is permitted to connect to the EEG monitoring module 1, the entity is in step 61 able to retrieve the unique identity of the EEG monitoring module 1 from the patient record. In step 62, the entity sends the Connection Information Request 31 containing the unique identity of the EEG monitoring module 1 to the EEG Module Connection Register 19a, and receives the current Connection information of the EEG monitoring module 1 in response. In step 63, the entity sets up the direct connection 33 from a computer 21, preferably using UDP hole punching, to the network connection handling element 43 in the EEG monitoring module 1 (in fig. 1 and 2) or in the personal communication device 13 connected to the EEG monitoring module 1 (in fig 3 and 4). In step 64, the entity terminates the direct connection when the exchange of data has been completed successfully.

Fig. 7 illustrates a flowchart of a method for maintaining the access data for an EEG monitoring module 1 according to an embodiment of the invention. In step 71, the network connection handling element 43 detects the presence of a new connection to the Internet 16; or the network connection handling element 43 detects the presence of a connection to the Internet 16 from the personal communication terminal 13 and a short range connection to the EEG monitoring module 1.

When sending the connection information notification 30 in step 72, the current connection information, including the IP address and port number; of the network connection handling element 43 is present as source address of the data packet, and the unique identity for the EEG monitoring module 1 is contained in the data packet as payload. The control server 19 extracts these two elements and updates the EEG Module Connection Register 19a accordingly.

These steps are repeated every time the network connection handling element 43 detects a change in the connection between the EEG monitoring module 1 and the Internet 16.

Fig. 8 illustrates a flowchart of a method for handling the establishment of the direct connection to the EEG monitoring module 1 according to an embodiment of the invention. When the network connection handling element 43 in step 73 receives a request for setting up a direct connection 33a, it starts verifying the validity of the connection in step 74. This may include verification of certificates. If the validity of the connection has been verified, the network connection handling element 43 confirms the establishment of the direct connection 33 in step 75.

In step 76, the computer 21 communicates to the network connection handling element 43 what kind of data that has to be exchanged - upload of logging data, download of settings etc. In step 77, the data requested is exchanged, and in step 78, data are handled as prescribed. New settings may overwrite earlier settings in the EEG monitoring module 1, and logging registers may be reset if their content has been uploaded. When the data has been exchanged and handled successfully, the direct connection session is terminated in step 79.

The number of electrodes has so far been identified as a pair of active electrodes operating in differential mode. However two or more active electrodes may be acting as sensing electrodes for measuring the electric potential difference relative to an active electrode acting as a common reference electrode. The electrodes will operate in a unipolar lead mode.

## Claims

1. A method for providing a network connection to a wearable EEG monitoring module via the Internet, wherein said wearable EEG monitoring module, which comprises a network connection handling element having a unique identity, is connected to the Internet via a gateway, and comprising the steps of:
- providing a plurality of patient records in a patient record server accessible over the Internet, each patient record including a unique identity for an associated wearable EEG monitoring module;
- detecting the presence of a wireless connection between said wearable EEG monitoring module and said gateway;
- communicating, triggered by the establishment of connection to the Internet via the gateway detected by the network connection handling element, the current connection information to a control server, wherein when sending the connection information, the current connection information of the network connection handling element is present as source address of the data packet embodying the connection information, and the unique identity for the EEG monitoring module is contained in the data packet;
- providing, in said control server, an EEG module connection register containing said unique identity of wearable EEG monitoring modules and corresponding communicated current connection information;
- requesting, from a computer device, said control server to provide the current connection information for said wearable EEG monitoring module by providing the associated unique identity, which has been obtained from said patient record server by accessing a patient record; and
- establishing, from said computer device, an internet connection to said wearable EEG monitoring module based on the current connection information retrieved from said control server.

2. The method according to claim 1, wherein an Internet enabled personal communication device and said wearable EEG monitoring module are connected for establishing a wireless connection, whereby the personal communication device becomes a gateway for said wearable EEG monitoring module to a patient record server over the Internet.

3. The method according to claim 1, and comprising retrieving, from said computer device, log data from said wearable EEG monitoring module via said gateway.

4. The method according to claim 1, and comprising automatically updating said EEG module connection register of the control server upon creation of new patient records in said patient record server.

5. The method according to claim 1, and comprising uploading current connection information to said control server upon detection of a new connection to the Internet.

6. The method according to claim 1, and comprising assigning in said EEG module connection register a unique identity to said wearable EEG monitoring module and mapping this unique identity to an IP address and a port number.

7. The method according to claim 2, wherein a medical care professional is retrieving data from and adjusting settings of said wearable EEG monitoring module during a remote EEG surveillance session by means of a computer program running on said computer device, wherein the computer program retrieves identification of said wearable EEG monitoring module from the patient record server and retrieves the associated Connection information from the EEG module connection register administered by the control server.

8. A system for providing a network connection for a computer device (21) to a wearable EEG monitoring module (1), which comprises a network connection handling element (43), said system having a gateway to the Internet (16), and comprising:
- a patient record server (25) accessible over the Internet, and providing an electronic medical record database (26), each patient record including a unique identity for an associated wearable EEG monitoring module;
- a control server (19) providing an EEG module connection register (19a) including said unique identity of EEG monitoring modules associated with current connection information;
- said network connection handling element (43) being adapted for detecting the presence of a wireless connection between said wearable EEG monitoring module (1) and said gateway, and for uploading current connection information to said control server (19) triggered by the establishment of connection to the Internet via the gateway, wherein when sending the connection information, the current connection information of the network connection handling element (43) is present as source address of the data packet embodying the connection information, and the unique identity for the EEG monitoring module is contained in the data packet; and
- wherein the control server (19) is adapted for updating the connection information in the EEG module connection register (19a) when receiving communicated connection information, and for providing the current connection information to a computer device (21) upon request; and
- wherein said computer device (21) is adapted for establishing an internet connection to said wearable EEG monitoring module (1) based on the current connection information requested from said control server (19).

9. The system according to claim 8, wherein said network connection handling element (43) is contained in said wearable EEG monitoring module (1).

10. The system according to claim 8, wherein said network connection handling element (43) is contained in an Internet enabled personal communication device (13), and wherein said Internet enabled personal communication device (13) and said wearable EEG monitoring module (1) are provided with respective transceivers for establishing a short range wireless connection, whereby the personal communication device (13) becomes said gateway for said wearable EEG monitoring module (1) to the Internet (16).

11. The system according to claim 10, wherein said network connection handling element (43) is adapted for detecting the presence of said short range wireless connection to said wearable EEG monitoring module (1), and for communicating said current connection information to said control server (19).

12. The system according to claim 8, wherein the computer device (21) is adapted for retrieving log data from said wearable EEG monitoring module (1) using said gateway.

13. The system according to claim 8, wherein said patient record server (25) and said control server (19) are two separate entities.

14. The system according to claim 8, wherein said patient record server (25) automatically updates said EEG module connection register (19a) of the control server (19) upon creation of new patient records.

15. The system according to claim 10, wherein said EEG module connection register (19a) of the control server (19) contains multiple connection information elements for each wearable EEG monitoring module present in the EEG module connection register (19a).

16. The system according to claim 8, wherein said network connection handling element (43) is adapted for communicating said current connection information to said control server (19) upon detection of a new connection to the Internet (16).

17. The system according to claim 8, wherein said EEG Module Connection Register (19a) of the control server (19) is adapted to assigning in said EEG module connection register (19a) a unique identity to said wearable EEG monitoring module (1) and mapping this unique identity to an IP address and a port number

18. A wearable EEG monitoring module (1) having a unique identity and being adapted to be connected to a computer device (21) via the Internet (16), and comprising a network connection handling element (43) being adapted to detecting the presence of a wireless connection between the wearable EEG monitoring module (1) and a gateway to the Internet (16), wherein the network connection handling element (43) is adapted to be triggered by the detection of the establishment of connection to the Internet via the gateway to communicate a current connection information for the wearable EEG monitoring module (1) to a control server (19), wherein when sending the connection information, the current connection information of the network connection handling element is present as source address of the data packet embodying the connection information, and the unique identity for the EEG monitoring module is contained in the data packet, whereby the computer device (21) is adapted to request the current connection information from said control server (19) based on the unique identity of the wearable EEG monitoring module (1), and for establishing an internet connection to the wearable EEG monitoring module (1).

## Patentansprüche

1. Verfahren zum Bereitstellen einer Netzwerkverbindung zu einem tragbaren EEG-Überwachungsmodul über das Internet, wobei das tragbare EEG-Überwachungsmodul, welches ein Netzwerkverbindungshandhabungselement mit einer eindeutigen Identität umfasst, über ein Gateway mit dem Internet verbunden ist, und umfassend die folgenden Schritte:
- Bereitstellen einer Vielzahl von Patientendatensätzen in einem Patientendatensatzserver, auf welchen über das Internet zugegriffen werden kann, wobei jeder Patientendatensatz eine eindeutige Identität für ein verknüpftes tragbares EEG-Überwachungsmodul beinhaltet;
- Erkennen des Vorhandenseins einer drahtlosen Verbindung zwischen dem tragbaren EEG-Überwachungsmodul und dem Gateway;
- Übertragen, ausgelöst durch die Herstellung einer Verbindung zu dem Internet über das Gateway, welche durch das Netzwerkverbindungshandhabungselement erkannt wurde, der aktuellen Verbindungsinformationen an einen Steuerserver, wobei beim Senden der Verbindungsinformationen die aktuellen Verbindungsinformationen des Netzwerkverbindungshandhabungselements als Quelladresse des Datenpakets, welches die Verbindungsinformationen verkörpert, vorhanden ist, und die eindeutige Identität für das EEG-Überwachungsmodul in dem Datenpaket enthalten ist;
- Bereitstellen, in dem Steuerserver, eines EEG-Modulverbindungsregisters, welches die eindeutige Identität von tragbaren EEG-Überwachungsmodulen und zugehörige übertragene aktuelle Verbindungsinformationen enthält;
- Anfordern, von einer Computervorrichtung, dass der Steuerserver die aktuellen Verbindungsinformationen für das tragbare EEG-Überwachungsmodul bereitstellt, durch Bereitstellen der verknüpften eindeutigen Identität, welche von dem Patientendatensatzserver durch Zugreifen auf einen Patientendatensatz erhalten wurde; und
- Herstellen, von der Computervorrichtung, einer Internetverbindung zu dem tragbaren EEG-Überwachungsmodul auf Basis der aktuellen Verbindungsinformationen, welche von dem Steuerserver abgerufen wurden.

2. Verfahren nach Anspruch 1, wobei eine internetfähige persönliche Übertragungsvorrichtung und das tragbare EEG-Überwachungsmodul zum Herstellen einer drahtlosen Verbindung verbunden sind, wobei die persönliche Übertragungsvorrichtung für das tragbare EEG-Überwachungsmodul zu einem Gateway zu einem Patientendatensatzserver über das Internet wird.

3. Verfahren nach Anspruch 1, und Abrufen, von Protokolldateien von dem tragbaren EEG-Überwachungsmodul von der Computervorrichtung über das Gateway umfassend.

4. Verfahren nach Anspruch 1, und automatisches Aktualisieren des EEG-Modulverbindungsregisters des Steuerservers bei Erstellung von neuen Patientendatensätzen in dem Patientendatensatzserver umfassend.

5. Verfahren nach Anspruch 1, und Hochladen aktueller Verbindungsinformationen auf den Steuerserver bei Erkennen einer neuen Verbindung zum Internet umfassend.

6. Verfahren nach Anspruch 1, und Zuweisen einer eindeutigen Identität zu dem tragbaren EEG-Überwachungsmodul und Zuordnen dieser eindeutigen Identität zu einer IP-Adresse und einer Portnummer in dem EEG-Modulverbindungsregister umfassend.

7. Verfahren nach Anspruch 2, wobei ein medizinisches Pflegefachpersonal während einer Remote-EEG-Überwachungssitzung von dem tragbaren EEG-Überwachungsmodul mithilfe eines Computerprogramms, welches auf der Computervorrichtung ausgeführt wird, Daten abruft und Einstellungen anpasst, wobei das Computerprogramm Identifikation des tragbaren EEG-Überwachungsmodul von dem Patientendatensatzserver abruft und die verknüpften Verbindungsinformationen von dem EEG-Modulverbindungsregister, welches von dem Steuerserver verwaltet wird, abruft.

8. System zum Bereitstellen einer Netzwerkverbindung für eine Computervorrichtung (21) zu einem tragbaren EEG-Überwachungsmodul (1), welches ein Netzwerkverbindungshandhabungselement (43) umfasst, wobei das System ein Gateway zum Internet (16) umfasst, und umfassend:
- einen Patientendatensatzserver (25), auf welchen über das Internet zugegriffen werden kann, und welcher eine Datenbank (26) für elektronische medizinische Datensätze bereitstellt, wobei jeder Patientendatensatz eine eindeutige Identität für ein verknüpftes tragbares EEG-Überwachungsmodul beinhaltet;
- einen Steuerserver (19), welcher ein EEG-Modulverbindungsregister (19a) bereitstellt, welcher die eindeutige Identität von EEG-Überwachungsmodulen beinhaltet, welche mit aktuellen Verbindungsinformationen verknüpft sind;
- wobei das Netzwerkverbindungshandhabungselement (43) zum Erkennen des Vorhandenseins einer drahtlosen Verbindung zwischen dem tragbaren EEG-Überwachungsmodul (1) und dem Gateway und zum Hochladen von aktuellen Verbindungsinformationen auf den Steuerserver (19), welches durch die Herstellung einer Verbindung zum Internet über das Gateway ausgelöst wird, adaptiert ist, wobei beim Senden der Verbindungsinformationen die aktuellen Verbindungsinformationen des Netzwerkverbindungshandhabungselements (43) als Quelladresse des Datenpakets, welches die Verbindungsinformationen verkörpert, vorhanden ist, und die eindeutige Identität für das EEG-Überwachungsmodul in dem Datenpaket enthalten ist; und
- wobei der Steuerserver (19) zum Aktualisieren der Verbindungsinformationen in dem EEG-Modulverbindungsregister (19a) beim Empfangen von übertragenen Verbindungsinformationen und auf Anfrage zum Bereitstellen der aktuellen Verbindungsinformationen für eine Computervorrichtung (21) adaptiert ist; und
- wobei die Computervorrichtung (21) zum Herstellen einer Internetverbindung zu dem tragbaren EEG-Überwachungsmodul (1) auf Basis der aktuellen Verbindungsinformationen, welche von dem Steuerserver (19) angefordert wurden, adaptiert ist.

9. System nach Anspruch 8, wobei das Netzwerkverbindungshandhabungselement (43) in dem tragbaren EEG-Überwachungsmodul (1) enthalten ist.

10. System nach Anspruch 8, wobei das Netzwerkverbindungshandhabungselement (43) in einer internetfähigen persönlichen Übertragungsvorrichtung (13) enthalten ist, und wobei die internetfähige persönliche Übertragungsvorrichtung (13) und das tragbare EEG-Überwachungsmodul (1) jeweils mit Sendern/Empfängern zum Herstellen einer drahtlosen Verbindung mit geringer Reichweite ausgestattet sind, wobei die persönliche Übertragungsvorrichtung (13) zu dem Gateway zum Internet (16) für das tragbare EEG-Überwachungsmodul (1) wird.

11. System nach Anspruch 10, wobei das Netzwerkverbindungshandhabungselement (43) zum Erkennen des Vorhandenseins der drahtlosen Verbindung mit geringer Reichweite zu dem tragbaren EEG-Überwachungsmodul (1) und zum Übertragen der aktuellen Verbindungsinformationen an den Steuerserver (19) adaptiert ist.

12. System nach Anspruch 8, wobei die Computervorrichtung (21) zum Abrufen von Protokolldateien von dem tragbaren EEG-Überwachungsmodul (1) unter Verwendung des Gateways adaptiert ist.

13. System nach Anspruch 8, wobei der Patientendatensatzserver (25) und der Steuerserver (19) zwei getrennte Einheiten sind.

14. System nach Anspruch 8, wobei der Patientendatensatzserver (25) das EEG-Modulverbindungsregister (19a) des Steuerservers (19) beim Erstellen von neuen Patientendatensätzen automatisch aktualisiert.

15. System nach Anspruch 10, wobei das EEG-Modulverbindungsregister (19a) des Steuerservers (19) mehrere Verbindungsinformationselemente für jedes tragbare EEG-Überwachungsmodul enthält, welches in dem EEG-Modulverbindungsregister (19a) vorhanden ist.

16. System nach Anspruch 8, wobei das Netzwerkverbindungshandhabungselement (43) zum Übertragen der aktuellen Verbindungsinformationen auf den Steuerserver (19) beim Erkennen einer neuen Verbindung zum Internet (16) adaptiert ist.

17. System nach Anspruch 8, wobei das EEG-Modulverbindungsregister (19a) des Steuerservers (19) zum Zuweisen einer eindeutigen Identität zu dem tragbaren EEG-Überwachungsmodul (1) und Zuordnen dieser eindeutigen Identität zu einer IP-Adresse und einer Portnummer in dem EEG-Modulverbindungsregister (19a) adaptiert ist.

18. Tragbares EEG-Überwachungsmodul (1), welches eine eindeutige Identität aufweist und adaptiert ist, um über das Internet (16) mit einer Computervorrichtung (21) verbunden zu werden, und ein Netzwerkverbindungshandhabungselement (43) umfassend , welches zum Erkennen des Vorhandenseins einer drahtlosen Verbindung zwischen dem tragbaren EEG-Überwachungsmodul (1) und einem Gateway zum Internet (16) adaptiert ist, wobei das Netzwerkverbindungshandhabungselement (43) adaptiert ist, durch das Erkennen der Herstellung einer Verbindung zum Internet über das Gateway ausgelöst zu werden, um eine aktuelle Verbindungsinformation für das tragbare EEG-Überwachungsmodul (1) an einen Steuerserver (19) zu kommunizieren, wobei beim Senden der Verbindungsinformationen die aktuellen Verbindungsinformationen des Netzwerkverbindungshandhabungselements als Quelladresse des Datenpakets, welches die Verbindungsinformationen verkörpert, vorhanden ist, und die eindeutige Identität für das EEG-Überwachungsmodul in dem Datenpaket enthalten ist, wobei die Computervorrichtung (21) adaptiert ist, um die aktuellen Verbindungsinformationen von dem Steuerserver (19) auf Basis der eindeutigen Identität des tragbaren EEG-Überwachungsmoduls (1) anzufordern, und zum Herstellen einer Internetverbindung zu dem tragbaren EEG-Überwachungsmodul (1).

## Revendications

1. Procédé pour fournir une connexion réseau à un module de surveillance EEG portable via Internet, dans lequel ledit module de surveillance EEG portable, qui comprend un élément de gestion de connexion réseau ayant une identité unique, est connecté à Internet via une passerelle, et comprenant les étapes suivantes :
- la fourniture d'une pluralité d'enregistrements de patient dans un serveur d'enregistrements de patient accessible sur Internet, chaque enregistrement de patient incluant une identité unique pour un module de surveillance EEG portable associé ;
- la détection de la présence d'une connexion sans fil entre ledit module de surveillance EEG portable et ladite passerelle ;
- la communication, déclenchée par l'établissement d'une connexion à Internet via la passerelle détectée par l'élément de gestion de connexion réseau, des informations de connexions actuelles à un serveur de commande, dans lequel, lors de l'envoi des informations de connexion, les informations de connexions actuelles de l'élément de gestion de connexion réseau sont présentes comme une adresse source du paquet de données incorporant les informations de connexion et l'identité unique pour le module de surveillance EEG est contenue dans le paquet de données ;
- la fourniture, dans ledit serveur de commande, d'un registre de connexion de modules EEG contenant ladite identité unique de modules de surveillance EEG portables et d'informations de connexions actuelles communiquées correspondantes ;
- la demande, à partir d'un dispositif informatique, audit serveur de commande de fournir les informations de connexions actuelles pour ledit module de surveillance EEG portable en fournissant l'identité unique associée, qui a été obtenue à partir dudit serveur d'enregistrements de patient en accédant à un enregistrement de patient ; et
- l'établissement, à partir dudit dispositif informatique, d'une connexion Internet audit module de surveillance EEG portable sur la base des informations de connexions actuelles récupérées à partir dudit serveur de commande.

2. Procédé selon la revendication 1, dans lequel un dispositif de communication personnel compatible Internet et ledit module de surveillance EEG portable sont connectés pour l'établissement d'une connexion sans fil, moyennant quoi le dispositif de communication personnel devient une passerelle pour ledit module de surveillance EEG portable vers un serveur d'enregistrements de patient sur Internet.

3. Procédé selon la revendication 1, et comprenant la récupération, à partir dudit dispositif informatique, de données de journal à partir dudit module de surveillance EEG portable via ladite passerelle.

4. Procédé selon la revendication 1, et comprenant la mise à jour automatique dudit registre de connexion de modules EEG du serveur de commande lors de la création de nouveaux enregistrements de patient dans ledit serveur d'enregistrements de patient.

5. Procédé selon la revendication 1, et comprenant le téléchargement d'informations de connexions actuelles vers ledit serveur de commande lors de la détection d'une nouvelle connexion à Internet.

6. Procédé selon la revendication 1, et comprenant l'attribution dans ledit registre de connexion de modules EEG d'une identité unique audit module de surveillance EEG portable et le mappage de cette identité unique à une adresse IP et un numéro de port.

7. Procédé selon la revendication 2, dans lequel un professionnel de soins médicaux récupère des données et ajuste des paramètres dudit module de surveillance EEG portable durant une session de surveillance EEG à distance au moyen d'un programme informatique exécuté sur ledit dispositif informatique, dans lequel le programme informatique récupère une identification dudit module de surveillance EEG portable à partir du serveur d'enregistrements de patient et récupère les informations de connexion associées à partir du registre de connexion de modules EEG administré par le serveur de commande.

8. Système pour fournir une connexion réseau pour un dispositif informatique (21) à un module de surveillance EEG portable (1), qui comprend un élément de gestion de connexion réseau (43), ledit système ayant une passerelle à Internet (16), et comprenant :
- un serveur d'enregistrements de patient (25) accessible sur Internet et fournissant une base de données d'enregistrements médicaux électroniques (26), chaque enregistrement de patient incluant une identité unique pour un module de surveillance EEG portable associé ;
- un serveur de commande (19) fournissant un registre de connexion de modules EEG (19a) incluant ladite identité unique de modules de surveillance EEG associés à des informations de connexions actuelles ;
- ledit élément de gestion de connexion réseau (43) étant adapté pour la détection de la présence d'une connexion sans fil entre ledit module de surveillance EEG portable (1) et ladite passerelle et pour le téléchargement des informations de connexions actuelles vers ledit serveur de commande (19) déclenché par l'établissement d'une connexion à Internet via la passerelle, dans lequel, lors de l'envoi des informations de connexion, les informations de connexions actuelles de l'élément de gestion de connexion réseau (43) sont présentes comme une adresse source du paquet de données incorporant les informations de connexion et l'identité unique pour le module de surveillance EEG est contenue dans le paquet de données ; et
- dans lequel le serveur de commande (19) est adapté pour la mise à jour des informations de connexion dans le registre de connexion de modules EEG (19a) lors de la réception d'informations de connexions communiquées et pour la fourniture des informations de connexions actuelles à un dispositif informatique (21) sur demande ; et
- dans lequel ledit dispositif informatique (21) est adapté pour l'établissement d'une connexion Internet audit module de surveillance EEG portable (1) sur la base des informations de connexions actuelles demandées à partir dudit serveur de commande (19).

9. Système selon la revendication 8, dans lequel ledit élément de gestion de connexion réseau (43) est contenu dans ledit module de surveillance EEG portable (1).

10. Système selon la revendication 8, dans lequel ledit élément de gestion de connexion réseau (43) est contenu dans un dispositif de communication personnel compatible Internet (13) et dans lequel ledit dispositif de communication personnel compatible Internet (13) et ledit module de surveillance EEG portable (1) sont munis d'émetteurs-récepteurs respectifs pour l'établissement d'une connexion sans fil à courte portée, moyennant quoi le dispositif de communication personnel (13) devient ladite passerelle pour ledit module de surveillance EEG portable (1) vers Internet (16).

11. Système selon la revendication 10, dans lequel ledit élément de gestion de connexion réseau (43) est adapté pour la détection de la présence de ladite connexion sans fil à courte portée audit module de surveillance EEG portable (1) et pour la communication desdites informations de connexions actuelles audit serveur de commande (19).

12. Système selon la revendication 8, dans lequel le dispositif informatique (21) est adapté pour la récupération de données de journal à partir dudit module de surveillance EEG portable (1) en utilisant ladite passerelle.

13. Système selon la revendication 8, dans lequel ledit serveur d'enregistrements de patient (25) et ledit serveur de commande (19) sont deux entités séparées.

14. Système selon la revendication 8, dans lequel ledit serveur d'enregistrements de patient (25) met à jour automatiquement ledit registre de connexion de modules EEG (19a) du serveur de commande (19) lors de la création de nouveaux enregistrements de patient.

15. Système selon la revendication 10, dans lequel ledit registre de connexion de modules EEG (19a) du serveur de commande (19) contient de multiples éléments d'informations de connexion pour chaque module de surveillance EEG portable présent dans le registre de connexion de modules EEG (19a).

16. Système selon la revendication 8, dans lequel ledit élément de gestion de connexion réseau (43) est adapté pour la communication desdites informations de connexions actuelles audit serveur de commande (19) lors de la détection d'une nouvelle connexion à Internet (16).

17. Système selon la revendication 8, dans lequel ledit registre de connexion de modules EEG (19a) du serveur de commande (19) est adapté pour l'attribution dans ledit registre de connexion de modules EEG (19a) d'une identité unique audit module de surveillance EEG portable (1) et le mappage de cette identité unique à une adresse IP et un numéro de port.

18. Module de surveillance EEG pouvant être porté (1) ayant une identité unique et étant adapté pour être connecté à un dispositif informatique (21) via Internet (16) et comprenant un élément de gestion de connexion réseau (43) qui est adapté pour la détection de la présence d'une connexion sans fil entre le module de surveillance EEG portable (1) et une passerelle à Internet (16), dans lequel l'élément de gestion de connexion réseau (43) est adapté pour être déclenché par la détection de l'établissement d'une connexion à Internet via la passerelle pour communiquer une information de connexion actuelle pour le module de surveillance EEG portable (1) à un serveur de commande (19), dans lequel, lors de l'envoi des informations de connexion, les informations de connexions actuelles de l'élément de gestion de connexion réseau sont présentes comme une adresse source du paquet de données incorporant les informations de connexion et l'identité unique pour le module de surveillance EEG est contenue dans le paquet de données, moyennant quoi le dispositif informatique (21) est adapté pour demander les informations de connexions actuelles à partir dudit serveur de commande (19) sur la base de l'identité unique du module de surveillance EEG portable (1) et pour l'établissement d'une connexion Internet au module de surveillance EEG portable (1).
